# FASCICULE DE BREVET EUROPEEN

(11) **EP 0 421 882 B1**
(45) Date de publication et mention de la délivrance du brevet: **30.11.1994**
(21) Numéro de dépôt: 90402754.7
(22) Date de dépôt: 04.10.1990
(51) Int. Cl.: C12P 7/18, C07C 31/18

(54) **Procédé de fabrication de xylitol et de produits riches en xylitol**
Verfahren zur Gewinnung von Xylit und an Xylit reichen Produkten
Process for preparing xylitol and products rich in xylitol

(30) Priorité: 04.10.1989 FR 8913003
(43) Date de publication de la demande: 10.04.1991
(73) Titulaire: Roquette Frères, F-62136 Lestrem (FR)
(72) Inventeur: Leleu, Jean-Bernard, F-62136 Lestrem (FR); Duflot, Pierrick, F-62136 Lestrem (FR); Caboche, Jean-Jacques, F-62400 Bethune (FR)
(74) Mandataire: Koch, Gustave

(56) Documents cités:
- EP-A- 0 403 392
- US-A- 519 446
- JOURNAL OF FERMENTATION TECHNOLOGY, vol. 63, no. 4, 1985; S. OHMOMO et al., pp. 331-335#
- CHEMICAL ABSTRACTS, vol. 77, no. 13, 25 septembre 1972, Columbus, OH (US); p. 306, no. 86674x#

## Description

L'invention a pour objet un procédé de fabrication de xylitol et de produits riches en xylitol.

Il est connu de préparer le xylitol par hydrogénation catalytique du D-xylose cristallisé ou de sirops riches en D-xylose, ce xylose cristallisé ou ces sirops riches en xylose étant eux-mêmes obtenus à partir de matières premières végétales telles que le bois de bouleau, les rafles de maïs, les coques de graines ou d'amandes.

Par hydrolyse acide de ces matières premières, dans des conditions extrêmes de température et de pression, on décompose les xylans --polymères du D-xylose-- en xylose dont l'application essentielle est, précisément, la fabrication --par hydrogénation-- du xylitol.

Ce procédé souffre toutefois de nombreux inconvénients dont les principaux sont:
- la faible teneur en xylans des matières premières, ce qui se traduit par de faibles rendements en xylitol (de 8 à 15% en poids de la matière première mise en oeuvre) et par la génération d'une quantité considérable de sous-produits pour lesquels il est difficile, voire illusoire de trouver une revalorisation et dont la mise au rebut s'avère très polluante,
- la présence dans les hydrolysats de ces matières premières d'autres sucres que le xylose, à savoir ceux du groupe comprenant le glucose, le mannose, le galactose et l'arabinose qui se transforment respectivement par hydrogénation en sorbitol, mannitol, galactitol et arabitol et dont les propriétés physiques sont proches de celles du xylitol, ce qui rend très difficile la séparation du xylitol; or, la présence de galactitol qui se trouve normalement dans les hydrolysats hydrogénés de ces matières végétales et qui cristallise en même temps que le xylitol lorsqu'on concentre les sirops, est indésirable dès lors que le xylitol est destiné à l'alimentation car ledit galactitol provoque la cataracte.

Le brevet français N° 2.009.331, qui décrit un procédé de fabrication de D-arabitol par fermentation du D-glucose, indique que le D-arabitol est une matière première importante pour préparer le D-xylose en passant par le D-xylulose; ce brevet reste toutefois muet sur les moyens susceptibles d'être mis en oeuvre pour transformer le D-xylulose en D-xylose et a fortiori en xylitol.

Il a aussi été proposé dès 1972, par la demande de brevet japonais N° 47-13707, de préparer du xylitol sans passer par l'étape intermédiaire du D-xylose, en hydrogénant directement le D-xylulose formé par fermentation aérobie du D-arabitol, lui-même obtenu par un procédé selon le susdit brevet français N° 2.009.331. L'inconvénient de ce procédé réside dans le fait que ladite hydrogénation ne fournit que 50% de xylitol pollué par la même quantité de D-arabitol, ce dernier rendant impossible, lorsqu'il est présent en de telles proportions, la cristallisation du xylitol; ce procédé ne permet donc pas de préparer le xylitol avec une pureté et un rendement élevés. En effet, seuls le D-xylose et son isomère optique, le L-xylose, fournissent 100% de xylitol par hydrogénation catalytique commme on le constate par dans US-B-519 446.

On a aussi essayé d'obtenir, par une hydrogénation stéréospécifique, le xylitol avec un rendement accru à partir du D-xylulose, et on a découvert récemment (J. Ferment. Technol., vol.66, No.1, 33-36, 1988) qu'il était possible de former le xylitol avec un rendement de 75% à partir de D-xylulose. Cette réduction ou hydrogénation stéréospécifique n'a cependant été obtenue qu'en 24 heures sur une solution à 2% de D-xylulose à l'aide de microorganismes immobilisés du genre Mycobacterium smegmatis. Il apparait clairement, au vu de ce qui précède, que ce procédé n'est pas envisageable industriellement du fait
- de la durée trop importante de la transformation du D-xylulose en xylitol,
- des concentrations utilisées qui sont trop faibles,
- des problèmes de toxicité provenant des mycobactéries mises en oeuvre du fait de leur caractère pathogène pour l'homme.

On sait également que l'isomérisation enzymatique du D-xylulose en D-xylose a été effectuée à titre expérimental par HOCHSTER et WATSON (National Research Council n° 3105 Ottawa, Canada) mais ceux-ci n'ont pas isolé le D-xylose du mélange isomérisé ainsi obtenu et n'ont pas proposé non plus son utilisation pour la fabrication du xylitol. En effet, cette isomérisation du D-xylulose en D-xylose n'est toujours que partielle et elle avait en outre été effectuée dans des conditions de température et surtout de concentration totalement incompatibles avec une mise en oeuvre industrielle.

De même, OHMOMO et ses collaborateurs ont proposé d'obtenir un sirop à très haute teneur en xylose en isomérisant un sirop de xylulose obtenu à partir d'arabitol, mais ils n'ont pas décrit la fabrication de xylitol à partir de ce sirop. La fabrication du xylose semblant seule les intéresser, ils ont également procédé pour obtenir les richesses maximales en xylose à l'isomérisation du xylulose à des matières sèches très faibles de 50 g/l en présence de grandes quantités d'ions activateurs et à pH très élevé, voisin de 9. Toutes ces conditions sont incompatibles avec un procédé industriel puisque les fortes dilutions obligent à traiter des volumes énormes, que les ions activateurs sont des polluants qu'il convient d'éliminer après l'isomérisation et surtout que le pH employé pour cette isomérisation, s'il conduit à des teneurs élevées en xylose, conduit également à la formation de produits de dégradation et d'épimérisation qui sont colorés et impossibles à éliminer du sirop isomérisé, rendant impossible une hydrogénation catalytique ultérieure que ces auteurs n'ont du reste pas envisagée.

Enfin, la demande de brevet européen EP-A-403 392 qui n'appartient à l'état de la technique que sous les conditions de l'article 54 (3) CBE, mentionne un procédé de production de xylitol plus performant mais plus complexe consistant à hydrogéner un sirop de xylose extrait par chromatographie à partir d'un sirop de xylose - xylulose lui-même obtenu par isomérisation d'un sirop de xylulose

Par conséquent, l'industriel a toujours besoin d'un procédé permettant de produire, à l'échelle industrielle, dans des conditions de salubrité et avec une pureté et un rendement suffisants, du xylitol à partir du D-xylulose, qui lui-même n'est obtenu qu'avec un rendement faible, voisin de 40%, à partir du D-glucose en passant par le D-arabitol.

L'invention a donc pour but, surtout, de fournir un procédé de préparation du xylitol à partir du D-xylulose, lui-même obtenu à partir du D-glucose via le D-arabitol, propre à aboutir au xylitol avec un rendement et une pureté suffisamment élevés pour que l'industriel puisse s'accomoder des nombreuses étapes conduisant depuis le D-glucose jusqu'au produit final recherché.

Et c'est à ce problème posé depuis près de vingt ans que la Société Demanderesse a eu le mérite d'apporter une solution en trouvant qu'il était possible d'obtenir le xylitol à l'état de haute pureté et dans des conditions économiques tout à fait avantageuses en hydrogénant catalytiquement un sirop de D-xylulose préalablement soumis à un traitement enzymatique d'isomérisation.

Il s'ensuit que le procédé de fabrication de xylitol et de produits riches en xylitol conforme à l'invention est caractérisé en ce qu'il consiste:
- à isomériser enzymatiquement un sirop de D-xylulose en un sirop contenant du D-xylose et du D-xylulose puis, sans en extraire le xylose,
- à hydrogéner catalytiquement ce sirop, conduisant ainsi à un sirop riche en xylitol,
ledit sirop riche en xylitol étant ou bien déshydraté, ou bien soumis à un traitement chromatographique ou à un traitement d'extraction par cristallisation.

Le D-xylulose peut être obtenu de façon connue en elle-même et notamment par oxydation microbiologique du D-arabitol, ce qui constitue d'ailleurs le procédé d'obtention préféré, ledit D-arabitol étant obtenu par fermentation aérobie du D-glucose.

Selon un mode de réalisation avantageux, le procédé de fabrication du xylitol conforme à l'invention est donc caractérisé par le fait que le D-xylulose servant de matière première est préparé par une succession de deux étapes comprenant:
- une fermentation aérobie d'un sirop de D-glucose à l'aide d'un microorganisme osmophile du genre Pichia, transformant le D-glucose en D-arabitol, et
- une fermentation aérobie du D-arabitol à l'aide d'un microorganisme producteur d'alcool déshydrogénase, du genre Acetobacter, Gluconobacter ou Klebsielle, propre à transformer le D-arabitol en D-xylulose et de fournir ainsi un sirop riche en D-xylulose.

La fermentation aérobie du D-glucose peut être remplacée par une variante consistant à passer par l'oxydation du D-glucose en acide D-gluconique qui, sous sa forme de sel de calcium, peut être décarboxylé selon la méthode dite de RUFF pour donner le D-arabinose, comme décrit dans le brevet américain N° 3.755.294. Le D-arabinose est ensuite hydrogéné de manière connue en elle-même pour fournir le D-arabitol.

En dépit de sa complexité apparente, le procédé conforme à l'invention permet, grâce à la combinaison particulière de ses étapes constitutives, d'obtenir le xylitol avec un rendement supérieur à 25% et pouvant atteindre 40% par rapport au D-glucose qui est une matière première abondante et de faible prix.

Par rapport aux procédés de l'art antérieur,
- les quantités de matière première à mettre en oeuvre et
- la pollution ainsi que le volume de sous-produits générés par la fabrication du xylitol
sont fortement diminués.

D'autres avantages résident dans le fait que
- les problèmes de logistique concernant la collecte de la matière première, le D-glucose, n'existent pas,
- le traitement de cette matière première peut être réalisé dans des équipements classiques qui n'ont pas à résister à des températures et pressions extrêmement élevées ni à des milieux corrosifs,
- le xylitol est obtenu exempt de galactitol et peut, par conséquent, être utilisé dans l'alimentation.

La Demanderesse a également découvert qu'il était possible, et ce contre toute attente, d'augmenter encore les performances du procédé de fabrication du xylitol exposé ci-dessus en soumettant à une nouvelle fermentation les eaux-mères de cristallisation du xylitol, riches en xylitol mais également en arabitol, à l'aide du même microorganisme producteur d'alcool déshydrogénase. Elle a découvert qu'un tel sirop fermenté, riche en D-xylulose mais contenant également une proportion importante de xylitol non transformé pouvait subir sans problème une isomérisation sous l'action de la xylose isomérase pour fournir un sirop riche en D-xylose et en xylitol et ce, malgré l'enseignement défavorable qu'avaient donné récemment IZUMORI et TUZAKI, "J. Ferment. Technol.", vol.66, No.1, 33-36 (1988); ces auteurs avaient conclu qu'il semblait bien que le xylitol soit un inhibiteur compétitif de la xylose isomérase.

Du fait des conclusions d'IZUMORI et de TUZAKI, la seule présence de xylitol dans des sirops de D-xylulose soumis à isomérisation devait donc faire craindre une forte influence négative sur la réaction d'isomérisation enzymatique.

Le sirop isomérisé, riche en D-xylose, xylitol et, dans une moindre mesure, en D-xylulose, peut être à nouveau hydrogéné pour fournir un sirop riche en xylitol duquel il est commode d'extraire le xylitol sous sa forme pure et cristallisée.

On peut également obtenir un xylitol un peu moins riche en fractionnant le sirop hydrogéné grâce à un procédé chromatographique tel que décrit dans le brevet français N° 2.344.514, par exemple, ce fractionnement chromatographique étant suivi d'un traitement de déshydratation ou de cristallisation. Le fractionnement du susdit sirop est d'ailleurs aisé car, contrairement à ce qui est exposé dans ce brevet en rapport avec le fractionnement d'hydrolysats de bois hydrogénés, le procédé selon l'invention ne fournit ni mannitol ni galactitol qui compliqueraient ce fractionnement. Les eaux-mères de cristallisation ou la fraction chromatographique riche en D-arabitol sont avantageusement recyclées à nouveau pour transformer le D-arabitol en D-xylulose.

Cette manière de faire permet d'atteindre, avec un rendement inespéré puisque proche de 100%, si l'on excepte les pertes inévitables dues aux purifications et concentrations intermédiaires souhaitables ou nécessaires dans la réalisation d'un tel procédé, une transformation quasi-totale du D-arabitol en xylitol.

L'invention sera encore mieux comprise à l'aide du complément de description qui suit, des exemples et du dessin ci-annexé, lesdits compléments de description, exemples et dessin étant relatifs à des modes de réalisation avantageux mais non limitatifs de l'invention.

Dans le susdit dessin,
- la figure 1 illustre schématiquement le procédé conforme à l'invention,
- les figures 2 et 3 illustrent schématiquement deux modes de réalisation préférés du susdit procédé.

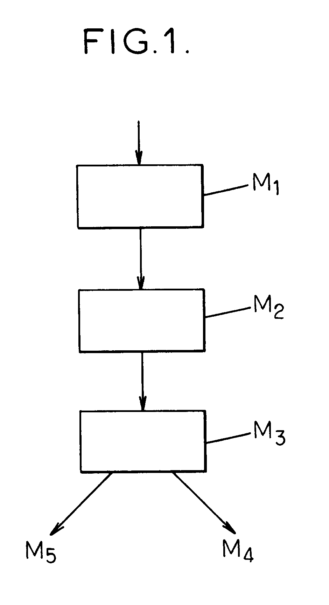

Sur la figure 1, on a représenté schématiquement:
- l'isomérisation enzymatique d'un sirop de D-xylulose en sirop riche en D-xylose en M₁,
- l'hydrogénation catalytique de ce sirop riche en D-xylose pour fournir un sirop riche en xylitol en M₂,
- l'extraction par cristallisation du xylitol à partir du sirop riche en xylitol en M₃, cette extraction fournissant du xylitol selon M₄ et des eaux-mères selon M₅.

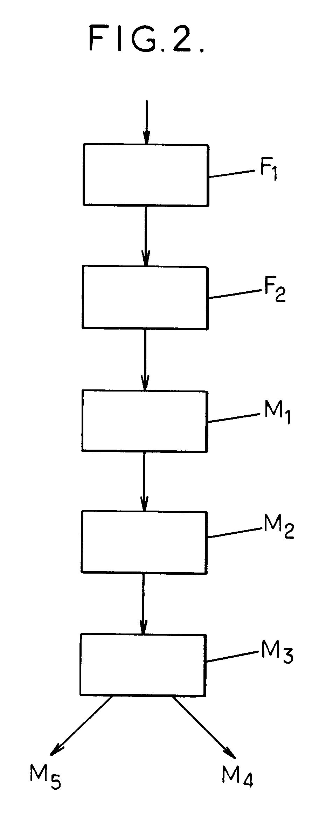

Sur la figure 2, on a représenté schématiquement:
- la fermentation aérobie d'un sirop de D-glucose à l'aide d'un microorganisme osmophile du genre Pichia transformant le D-glucose en D-arabitol en F₁,
- la fermentation aérobie du D-arabitol à l'aide d'un microorganisme producteur d'alcool déshydrogénase du genre Acetobacter transformant le D-arabitol en D-xylulose en F₂,
- en M₁, M₂, M₃, M₄ et M₅, les étapes illustrées par la figure 1.

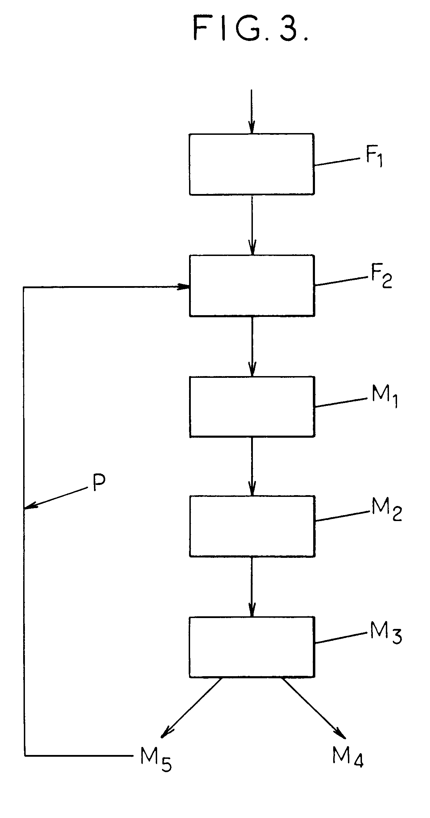

Sur la figure 3, on a représenté schématiquement:
- la succession des étapes F₁, F₂, M₁, M₂, M₃, M₄ et M₅ selon la figure 2,
- le traitement des eaux-mères de la cristallisation (M₅) du xylitol obtenu en M₃ par une nouvelle succession des étapes F₂, M₁, M₂, M₃, M₄ et M₅ après recyclage en F₂ suivant P.

Pour la fermentation du glucose, on peut avoir recours à un milieu de culture présentant la composition suivante:

| | |
|---|---|
| - dextrose | 150 à 200 g/l |
| - azote organique (sous forme de corn-steep ou d'extrait de levure | 2 à 4 g/l (N x 6,25) |
| - KH₂PO₄ | 1 à 3 g/l |
| - MgSO₄, 7H₂O | 1 à 2 g/l |

milieu qui est introduit dans un fermenteur, puis stérilisé et inoculé à l'aide de 10% environ d'une culture de 24 heures d'un microorganisme du genre Pichia, par exemple de la souche Pichia Ohmeri n°20.209 conservée à l'A.T.C.C. (ou d'une souche de Pichia farinosa), cette culture ayant été réalisée sur un milieu constitué par exemple comme suit:

| | |
|---|---|
| - dextrose | 50 g/l |
| - extrait de levure | 10 g/l |
| - KH₂PO₄ | 3 g/l |
| - MgSO₄, 7H₂O | 1 g/l. |

La fermentation est poursuivie à une température voisine de 30°C durant 80 à 100 heures sous une aération correspondant à 1 à 1,5 volume d'air/volume de culture/minute, et à un pH compris entre 4 et 6, de préférence voisin de 4,5, avantageusement maintenu par de l'ammoniaque, ce grâce à quoi on obtient généralement une teneur en D-arabitol de 65 à 90 g/l, cet arabitol représentant de 70 à 85% des matières présentes dans le milieu de culture au terme de cette fermentation, les impuretés sucrées étant surtout constituées par du xylitol formé de façon parallèle à l'arabitol et par du glucose non fermenté.

Le rendement en D-arabitol par rapport au glucose mis en oeuvre est d'environ 45 à 50%.

Le contenu entier du fermenteur (moût de fermentation riche en D-arabitol) peut être alors stérilisé de manière à détruire la levure puis être ensuite ensemencé par un inoculum (10% environ) d'une culture d'acetobacter suboxydans.

Il peut être avantageux de soumettre le mout de fermentation riche en D-arabitol à une purification par centrifugation ou filtration avant ensemencement par l'acetobacter.

De même, cette purification peut être complétée par une déminéralistion, une décoloration puis une concentration permettant de cristalliser du D-arabitol avec une richesse voisine de 98% et un rendement de 40% environ par rapport au glucose mis en oeuvre.

Le D-arabitol obtenu à l'étape précédente peut être fermenté à une concentration avantageusement comprise entre 65 et 220 g/l dans un milieu de culture contenant de l'azote organique et des sels minéraux qu'il convient d'ajouter de façon d'autant plus importante que l'on a poussé la purification de l'arabitol. Les proportions adéquates sont facilement déterminables par l'homme du métier et seront telles que l'on obtienne environ:

| | |
|---|---|
| azote organique | 1 à 2 g/l |
| KH₂PO₄ | 1 g/l |
| MgSO₄, 7H₂O | 1 g/l. |

La préculture est avantageusement une culture d'acetobacter suboxydans cultivé 20 heures environ sur un milieu de la composition suivante:

| | |
|---|---|
| - arabitol | 50 g/l |
| - sorbitol | 2 g/l |
| - extrait de levure | 2 g/l |
| - KH₂PO₄ | 0,2 g/l |
| - MgSO₄, 7H₂O | 0,2 g/l |
| - CaCO₃ | 5 g/l. |

La fermentation du D-arabitol est conduite à une température de 20 à 40°C, sous une aération correspondant à 1 à 1,5 volume d'air/volume de culture/minute, à un pH de 4,0 à 6,0 et durant un temps généralement compris entre 24 et 48 heures, terme au bout duquel 95% du D-arabitol présent dans le moût de culture sont convertis pratiquement quantitativement en D-xylulose. Il est avantageux de ne pas poursuivre la fermentation au-delà de ce moment car il se produirait ensuite des produits de dégradation ou d'interconversion des sucres ou de leurs dérivés, produits dont l'accumulation pourrait nuire à la suite des opérations.

Les eaux-mères de la cristallisation du xylitol obtenu selon le procédé de l'invention peuvent avantageusement subir cette fermentation par l'acetobacter puis-qu'elles sont très riches en D-arabitol.

On constate que la présence de xylitol en forte quantité par rapport à l'arabitol n'entrave pas cette fermentation dans la mesure où la concentration en arabitol + xylitol demeure inférieure à environ 220 g/litre.

Dans ce cas, seul le D-arabitol est oxydé en D-xylulose par l'acetobacter, le xylitol n'étant pas oxydé en xylulose mais demeurant intransformé dans la mesure où l'on arrête cette fermentation lorsque le D-arabitol ne représente plus que 1 à 5% des sucres présents.

La composition glucidique des moûts de fermentation obtenus par cette action de l'acetobacter est variable et est fonction de la pureté de l'arabitol, le xylitol présent dans les moûts n'étant pas fermentescible, ni transformé dans les conditions décrites.

Ce moût de fermentation peut être purifié de manière connue en elle-même par filtration, décoloration au charbon actif et déminéralisation, puis concentré avant d'être soumis à l'étape d'isomérisation.

Il peut être obligatoire de recourir à la susdite purification si l'isomérisation est effectuée en continu à l'aide d'une enzyme immobilisée dans un réacteur à effet piston; cette purification est superflue dans le cas où l'on procède à une isomérisation en lot à enzyme perdue et de façon discontinue.

On peut utiliser pour l'étape d'isomérisation une glucose isomérase commerciale du type de celles mises en oeuvre pour la fabrication des sirops de maïs à haute teneur en fructose, à savoir par exemple:
- celle qui est connue sous la marque SPEZYME® et qui est commercialisée par Genencor,
- celle qui peut être obtenue selon le brevet français N° 2.353.562 dont la Demanderesse est titulaire.

De préférence, la quantité d'enzyme mise en oeuvre est telle que l'équilibre de la réaction soit atteint en 10 minutes à 48 heures selon la technologie continue ou discontinue mise en oeuvre; la présence d'un agent protecteur de l'enzyme, tel que le bisulfite de soude et/ou un sel de magnésium est souhaitable.

L'isomérisation est conduite à une température de 40 à 80°C et à un pH de préférence compris entre 6,0 et 8,5.

D'une façon générale, les paramètres de l'étape d'isomérisation sont choisis de façon telle que 50 à 75% environ du D-xylulose présent dans le sirop isomérisé soient convertis en D-xylose.

A l'issue de l'étape d'isomérisation, la composition glucidique du sirop isomérisé obtenu varie bien entendu selon la composition du sirop soumis à isomérisation. Elle est d'autant moins riche en xylose que le sirop soumis à l'isomérisation était plus riche en xylitol.

Il a été constaté avec surprise que la présence du xylitol formé de façon parallèle à l'arabitol lors de la fermentation du glucose mais essentiellement causée par le recyclage des eaux-mères au niveau de l'étape d'oxydation microbiologique à l'aide de l'acetobacter dans le mode de mise en oeuvre préféré de l'invention, n'affectait pas le fonctionnement de la glucose isomérase puisque les taux maximum de conversion du D-xylulose en D-xylose sont identiques (voisins de 75%) aux taux obtenus antérieurement dans l'isomérisation du xylose pur et ce fait était complètement inattendu, comme il a déjà été expliqué.

Le sirop riche en D-xylose obtenu après isomérisation est purifié par déminéralisation et est ensuite soumis à l'hydrogénation catalytique à l'aide de catalyseurs au ruthénium ou au nickel de Raney.

De préférence, l'étape d'hydrogénation est effectuée avec un catalyseur au nickel de Raney, à une pression d'hydrogène supérieure à 20 bars et comprise de préférence entre 40 et 70 bars, à une température de 100 à 150°C et à une concentration de 20 à 60%. L'hydrogénation est poursuivie jusqu'à ce que la teneur en sucres réducteurs du sirop hydrogéné soit inférieure à 2%, de préférence inférieure à 1% et plus préférentiellement encore inférieure à 0,5% (la teneur en sucres réducteurs étant définie en poids d'équivalent dextrose par rapoprt à la matière sèche).

Le sirop hydrogéné ainsi obtenu, très riche en xylitol, est ensuite filtré pour enlever le catalyseur, puis il est déminéralisé.

La teneur en xylitol de ce sirop hydrogéné, variable selon la composition du sirop soumis à l'hydrogénation, est généralement supérieure à 80% et peut dépasser 90%.

Ce sirop peut être déshydraté et utilisé tel quel pour des applications techniques.

Un produit plus pur peut être obtenu en soumettant ledit sirop à un fractionnement chromatographique, par exemple en utilisant l'enseignement du brevet français N° 2.344.515; le produit ainsi obtenu est soumis à un traitement de déshydratation ou de cristallisation.

Pour obtenir du xylitol pur, on concentre le susdit sirop puis on le refroidit de façon à permettre la cristallisation du xylitol en un ou plusieurs jets.

De préférence, le sirop est concentré jusqu'à une teneur en matières sèches voisine de 84% et qui peut être d'autant plus basse que la richesse en xylitol du sirop est élevée. Le sirop de xylitol concentré est ensuite lentement refroidi après l'avoir additionné de germes cristallins de xylitol. On peut également ajouter les germes cristallins durant la concentration du sirop de xylitol. De préférence, le sirop est refroidi jusqu'à une température voisine de 20°C et ce sous agitation lente.

Lorsque la cristallisation est achevée, ce qui se produit généralement au terme d'une période de 20 à 90 heures, la masse cristallisée ainsi formée est essorée et les cristaux de xylitol sont lavés puis séchés.

Ce xylitol a généralement une pureté supérieure à 97%.

Les eaux-mères de la cristallisation, riches en xylitol mais également en arabitol, sont généralement épuisées en un ou plusieurs jets de cristallisation jusqu'à une teneur en xylitol qui peut être aussi basse que 50, voire 45%.

Selon un mode de réalisation préféré du procédé selon l'invention, ces eaux-mères sont soumises à nouveau aux étapes d'oxydation microbienne, d'isomérisation enzymatique, d'hydrogénation catalytique et de cristallisation.

On peut également en extraire du xylitol par un procédé chromatographique.

### EXEMPLE 1

Un sirop de D-xylulose obtenu à partir de D-glucose via le D-arabitol par double fermentation et sans cristalliser ce dernier, a été purifié par filtration, décoloration et déminéralisation, puis a été concentré à une matière sèche de 25%.

Ce sirop avait la composition suivante:

| | |
|---|---|
| D-arabitol | 1 % |
| D-xylulose | 95 % |
| D-xylitol | 3 % |
| divers | 1 %. |

Il a été percolé à une température de 65°C sur une colonne de glucose isomérase immobilisée de marque SPEZYME après avoir été ajusté à un pH de 7,7 par du carbonate de sodium et après addition de 0,4 g/l de bisulfite de sodium et 1 g/l de chlorure de magnésium.

Ce sirop a été percolé à un débit de 3 volumes de sirop/volume de colonne/heure.

On a obtenu par cette réaction d'isomérisation un sirop isomérisé de la composition suivante:

| | |
|---|---|
| D-arabitol | 1 % |
| D-xylulose | 25 % |
| D-xylose | 70 % |
| D-xylitol | 3 % |
| divers | 1 %. |

On a constaté avec surprise que la présence du xylitol formé de façon parallèle à l'arabitol lors de l'étape de fermentation du glucose et non transformé au moment de la fermentation de l'arabitol en xylulose, ne perturbe pas le fonctionnement de la réaction d'isomérisation puisque le taux de xylose obtenu par rapport au xylulose présent dans le sirop avant isomérisation est proche de 75%, soit identique aux taux obtenus antérieurement lors de l'isomérisation de xylose pur.

Ce sirop riche en xylose a été déminéralisé puis hydrogéné sous une pression d'hydrogène de 50 bars en présence de nickel de Raney et à une température de 120°C. Après 3 heures d'hydrogénation, ce sirop présentait une teneur en sucres réducteurs de 0,1%.

Sa composition était alors la suivante:

| | |
|---|---|
| D-arabitol | 13,5 % |
| xylitol | 85,5 % |
| divers | 1 %. |

Ce sirop a été déminéralisé puis concentré à une matière sèche de 84% et refroidi en 30 heures de 60 à 25°C sous agitation lente après avoir été ensemencé par du xylitol cristallisé.

La masse cristallisée obtenue a été essorée puis lavée et on a obtenu du xylitol cristallisé pur avec un rendement de 50% par rapport au poids sec de sirop de xylulose mis en oeuvre.

Les eaux-mères de la cristallisation ont été à nouveau concentrées à une matière sèche de 86% et ont été à nouveau soumises à cristallisation. Elles ont fourni un deuxième jet de xylitol cristallisé pur portant ainsi le rendement de la cristallisation à 70%.

Les eaux-mères de ce deuxième jet avaient la composition suivante:

| | |
|---|---|
| D-arabitol | 45 % |
| xylitol | 52 % |
| divers | 3 %. |

Le xylitol cristallisé a été ainsi obtenu avec un rendement de 28% par rapport au glucose mis en oeuvre.

### EXEMPLE 2

Dans un fermenteur d'une capacité totale de 10 m³, on a introduit:
1200 kg de dextrose cristallisé monohydrate,
16 kg d'extrait de levure,
8 kg de KH₂PO₄,
8 kg de MgSO₄, 7H₂O
7000 litres d'eau.

Après stérilisation du milieu de culture et refroidissement à 30°C, on a inoculé ce fermenteur à l'aide de 800 litres d'une préculture de Pichia Ohmeri ATCC 20.209 telle que décrite dans le brevet français N° 2.009.331, préculture agée de 24 heures.

L'aération a été poursuivie durant toute la durée de la transformation du glucose en arabitol, soit durant 90 heures avec un débit de 130 Nm³/heure et le pH a été maintenu, par addition d'ammoniaque, à une valeur de 4,5.

Le moût de fermentation ainsi obtenu a été filtré pour en éliminer la levure, puis il a été décoloré par du charbon activé et déminéralisé sur des résines échangeuses d'ions du type résine cationique forte suivie d'une résine anionique forte.

Le sirop incolore obtenu avait la composition suivante:

| | |
|---|---|
| arabitol | 95 % |
| xylitol | 3 % |
| glucose | 1 % |
| divers | 1 %. |

Dans un évaporateur-cristallisoir, on a concentré ce sirop purifié jusqu'à l'obtention d'une masse épaisse de cristaux de D-arabitol, puis on a essoré et lavé ces cristaux.

Les eaux-mères ont été à nouveau concentrées pour en extraire un deuxième jet de D-arabitol et on a obtenu celui-ci sous forme de cristaux blancs d'une richesse de 99% avec un rendement de 35% par rapport au glucose mis en oeuvre.

On a mélangé dans un fermenteur de 10 m³, 800 kg de cristaux de D-arabitol obtenu à l'étape précédente avec 800 kg de matière sèche d'eaux-mères de xylitol obtenues à l'exemple 1, puis on a introduit dans ce fermenteur:
16 kg d'extrait de levure,
8 kg de KH₂PO₄,
8 kg de MgSO₄, 7H₂O
6800 litres d'eau.

La composition sucrée de ce moût de culture était la suivante:

| | |
|---|---|
| arabitol | 72,5 % |
| xylitol | 26 % |
| divers | 1,5 %. |

Après stériliation et refroidissement, ce fermenteur a été ensemencé à l'aide de 600 litres d'une préculture de 24 heures d'acetobacter suboxydans cultivée sur un milieu de la composition suivante:

| | |
|---|---|
| arabitol | 50 g/l |
| sorbitol | 2 g/l |
| extrait de levure | 2 g/l |
| KH₂PO₄ | 0,2 g/l |
| MgSO₄, 7 H₂O | 0,2 g/l. |

Après 24 heures de fermentation sous une aération de 1 volume/volume/minute, on a porté le contenu du fermenteur à une température de 60°C, on y a ajouté 3,2 kg de bisulfite de sodium et on a ajusté le pH à 7,7 à l'aide de carbonate de sodium. On y a ensuite ajouté 16 litres de glucose isomérase liquide commercialisée par la Société Gist-Brocadés, titrant 4000 unités internationales par ml. Après 6 heures d'isomérisation dans ces conditions, la composition du milieu de culture qui était avant l'isomérisation de:

| | |
|---|---|
| xylulose | 71,5 % |
| xylitol | 27 % |
| divers | 1,5 % |

s'est établie après l'isomérisation à

| | |
|---|---|
| xylulose | 18,6 % |
| xylose | 52,9 % |
| xylitol | 27 % |
| divers | 1,5 %. |

On constate donc que la présence de très fortes concentrations de xylitol et des impuretés de la fermentation n'a pas gêné l'isomérisation du xylulose en xylose puisque le taux de transformation a été proche de 75%.

Ce sirop a été filtré, décoloré et déminéralisé puis, après avoir été concentré à une matière sèche de 40%, il a été hydrogéné dans les conditions décrites à l'exemple 1.

On a obtenu un sirop de xylitol de la composition suivante:

| | |
|---|---|
| xylitol | 89,2 % |
| arabitol | 9,3 % |
| divers | 1,5 %. |

Ce sirop peut être aisément cristallisé selon le procédé qui a été décrit à l'exemple 1.

### EXEMPLE 3

Les eaux-mères de deuxième jet de cristallisation du xylitol obtenu à l'exemple 1 ont été diluées à une matière sèche de 200 g/l puis ont été additionnées d'extrait de levure et de sels minéraux, comme décrit à l'exemple 2.

Après fermentation par l'acetobacter suboxydans dans les conditions décrites à propos de l'exemple 2, on a obtenu un moût de culture dont la composition sucrée était la suivante:

| | |
|---|---|
| xylulose | 43,7 % |
| xylitol | 53 % |
| divers | 3,3 %. |

On a procédé à l'isomérisation de ce moût de culture comme dans l'exemple 2 et on a obtenu un moût isomérisé dont la composition sucrée était la suivante:

| | |
|---|---|
| xylose | 32,4 % |
| xylulose | 11,3 % |
| xylitol | 53 % |
| divers | 3,3 %. |

On constate encore une fois que, de manière absolument surprenante, l'isomérisation du D-xylulose en D-xylose n'a pas été entravée par la présence de xylitol qui constitue pourtant le plus important composant du moût soumis à l'isomérisation.

Le moût isomérisé a été filtré puis décoloré et déminéralisé, puis il a été concentré à une matière sèche de 40% en vue de l'hydrogénation qui a été réalisée dans les conditions décrites à l'exemple 1.

Le sirop hydrogéné ainsi obtenu avait la composition suivante:

| | |
|---|---|
| xylitol | 91 % |
| arabitol | 5,7 % |
| divers | 3,3 %. |

Trois jets de cristallisation ont permis d'extraire sous forme de xylitol pur et cristallisé 80% de la matière de ce sirop soumis à la cristallisation, portant ainsi à 37% le rendement de 28% obtenu à l'exemple 1 en xylitol cristallisé par rapport au glucose mis en oeuvre.

Il résulte des valeurs numériques indiquées dans ces exemples que grâce au procédé conforme à l'invention, on obtient le xylitol cristallisé à partir du D-xylulose et donc du D-glucose avec un rendement qu'aucun des procédés empruntant la voie du xylulose ne permettait d'obtenir, ce rendement étant par ailleurs bien supérieur à ceux que permettaient d'obtenir les procédés d'hydrolyse de matières premières végétales contenant des xylans.

## Revendications

1. Procédé de fabrication de xylitol et de produits riches en xylitol, caractérisé en ce qu'il consiste:
- à isomériser enzymatiquement un sirop de D-xylulose en un sirop contenant du D-xylose et du D-xylulose puis, sans en extraire le xylose,
- à hydrogéner catalytiquement ce sirop, conduisant ainsi à un sirop riche en xylitol,
ledit sirop riche en xylitol étant ou bien déshydraté, ou bien soumis à un traitement chromatographique ou à un traitement d'extraction par cristallisation.

2. Procédé selon la revendication 1, caractérisé par le fait que le sirop de D-xylulose de départ est obtenu par fermentation aérobie de D-arabitol.

3. Procédé selon la revendication 2, caractérisé par le fait que le D-arabitol de départ est obtenu par fermentation aérobie de D-glucose.

4. Procédé selon la revendication 2, caractérisé par le fait que le D-arabitol est obtenu par hydrogénation du D-arabinose.

5. Procédé de fabrication du xylitol caractérisé par le fait qu'il comprend:
- une étape de transformation microbiologique de D-arabitol en D-xylulose à l'aide de microorganismes producteurs d'alcool déshydrogénase,
- une étape de transformation enzymatique du D-xylulose de l'étape précédente en D-xylose à l'aide d'une glucose-isomérase,
- une étape d'hydrogénation catalytique du sirop de D-xylose de l'étape précédente en un sirop riche en xylitol,
- une étape de récupération par cristallisation du xylitol à partir du sirop riche en xylitol de l'étape précédente et séparation de ces cristaux de leurs eaux-mères.

6. Procédé de fabrication du xylitol selon la revendication 5, caractérisé par le fait que le D-arabitol est obtenu par transformation microbiologique du D-glucose à l'aide de levures osmophiles.

7. Procédé selon la revendication 5, caractérisé par le fait que les eaux-mères dont a été retiré le xylitol cristallisé sont recyclées au niveau de l'étape de transformation microbiologique du D-arabitol en D-xylulose.

8. Procédé selon la revendication 5, caractérisé par le fait que l'on utilise comme matière première les eaux-mères dont a été retiré le xylitol cristallisé.

## Claims

1. Process for manufacturing xylitol and xylitol-rich products, which process consists of:
- enzymatically isomerizing a D-xylulose syrup into a syrup containing D-xylose and D-xylulose and then, without extracting the xylose,
- catalytically hydrogenating this syrup, resulting thus in a syrup rich in xylitol,
said xylitol-rich syrup being either dehydrated, or subjected to chromatographic treatment or to a treatment of extraction by crystallization.

2. Process according to claim 1, wherein the starting D-xylulose syrup is obtained by aerobic fermentation of D-arabitol.

3. Process according to claim 2, wherein the starting D-arabitol is obtained by aerobic fermentation of D-glucose.

4. Process according to claim 2, wherein the D-arabitol is obtained by hydrogenation of D-arabinose.

5. Process for manufacturing xylitol said process comprising:
- a step of micro biological conversion of D-arabitol into D-xylulose by means of microorganisms producing alcohol dehydrogenase,
- a step of enzymatic conversion of D-xylulose from the preceding step into D-xylose by means of glucose-isomerase,
- a step of catalytic hydrogenation of the D-xylose syrup from the preceding step into a syrup rich in xylitol,
- a step of recovery by crystallization of the xylitol from xylitol-rich syrup from the preceding step and separation of these crystals from their mother liquors.

6. Process for manufacturing xylitol according to claim 5, wherein the D-arabitol is obtained by microbiological conversion of D-glucose by means of osmophilic yeasts.

7. Process according to claim 5, wherein the mother liquors from which crystalline xylitol has been withdrawn are recycled at the level of the microbiological conversion step of the D-arabitol into D-xylulose.

8. Process according to claim 5, wherein there is used as a raw material mother liquors from which the crystalline xylitol has been extracted.

## Patentansprüche

1. Verfahren zur Herstellung von Xylit und an Xylit reichen Produkten, dadurch gekennzeichnet, daß es darin besteht:
- einen Sirup von D-Xylulose enzymatisch zu isomerisieren zu einem Sirup, der D-Xylose und D-Xylulose enthält, und dann, ohne daraus die Xylose zu extrahieren,
- diesen Sirup katalytisch zu hydrieren, was so zu einem Sirup, reich an Xylit, führt,
wobei dieser Sirup, reich an Xylit, entweder dehydratisiert oder einer chromatographischen Behandlung oder einer Extraktionsbehandlung durch Kristallisation unterworfen wird.

2. Verfahren gemäß Anspruch 1, dadurch gekennzeichnet, daß der Ausgangs-D-Xylulose-Sirup durch aerobe Fermentation von D-Arabit erhalten wird.

3. Verfahren gemäß Anspruch 2, dadurch gekennzeichnet, daß der Ausgangs-D-Arabit durch aerobe Fermentation von D-Glucose erhalten wird.

4. Verfahren gemäß Anspruch 2, dadurch gekennzeichnet, daß der D-Arabit durch Hydrierung von D-Arabinose erhalten wird.

5. Verfahren zur Herstellung von Xylit, dadurch gekennzeichnet, daß es umfaßt:
- eine mikrobiologische Umwandlungsstufe von D-Arabit zu D-Xylulose mittels Alkoholdehydrogenase erzeugenden Mikroorganismen,
- eine enzymatische Umwandlungsstufe von D-Xylulose der vorhergehenden Stufe zu D-Xylose mittels einer Glucose-Isomerase,
- eine Stufe der katalytischen Hydrierung des D-Xylose-Sirups der vorhergehenden Stufe zu einem Sirup, reich an Xylit,
- eine Stufe der Gewinnung durch Kristallisation des Xylits aus dem Sirup, reich an Xylit, der vorhergehenden Stufe und Abtrennung dieser Kristalle aus ihren Mutterlaugen.

6. Verfahren zur Herstellung von Xylit nach Anspruch 5, dadurch gekennzeichnet, daß der D-Arabit erhalten wird durch mikrobiologische Umwandlung der D-Glucose mittels osmophiler Hefen.

7. Verfahren gemäß Anspruch 5, dadurch gekennzeichnet, daß die Mutterlaugen, aus denen man den kristallisierten Xylit abgezogen hat, auf der Stufe der mikrobiologischen Umwandlung von D-Arabit zu D-Xylulose recycliert werden.

8. Verfahren gemäß Anspruch 5, dadurch gekennzeichnet, daß man als Ausgangsmaterial die Mutterlaugen verwendet, von denen man den kristallisierten Xylit abgezogen hat.
